(19) Europäisches Patentamt

European Patent Office

Office européen des brevets

(11) Veröffentlichungsnummer : **0 209 021 B1**

# (12) EUROPÄISCHE PATENTSCHRIFT

(45) Veröffentlichungstag der Patentschrift :
**23.01.91 Patentblatt 91/04**

(21) Anmeldenummer : **86109184.1**

(22) Anmeldetag : **04.07.86**

(51) Int. Cl.⁵ : **C07D 201/12**

---

(54) **Verfahren zur Gewinnung von Caprolactam durch Spaltung von Oligomeren des Caprolactams.**

---

Die Akte enthält technische Angaben, die nach
dem Eingang der Anmeldung eingereicht
wurden und die nicht in dieser Patentschrift
enthalten sind.

(30) Priorität : **09.07.85 DE 3524394**

(43) Veröffentlichungstag der Anmeldung :
**21.01.87 Patentblatt 87/04**

(45) Bekanntmachung des Hinweises auf die
Patenterteilung :
**23.01.91 Patentblatt 91/04**

(84) Benannte Vertragsstaaten :
**BE DE FR GB IT NL**

(56) Entgegenhaltungen :
**EP-A- 0 046 183**
**DE-A- 1 953 153**
**DE-A- 2 641 381**

(73) Patentinhaber : **BASF Aktiengesellschaft**
**Carl-Bosch-Strasse 38**
**D-6700 Ludwigshafen (DE)**

(72) Erfinder : **Fuchs, Hugo, Dr.**
**Egellstrasse 28**
**D-6700 Ludwigshafen (DE)**
Erfinder : **Brand, Uwe**
**Rheingoldstrasse 12**
**D-6840 Lampertheim (DE)**
Erfinder : **Buchaeckert, Helmut**
**Feldwiesenstrasse 10**
**D-6737 Boehl-Iggelheim (DE)**

## Beschreibung

Aus der DE-OS 30 30 735 ist ein Verfahren zur Gewinnung von Caprolactam durch Spaltung von Oligomeren des Caprolactams, wobei man die Oligomeren flüssig oder in festem Zustand in eine Aluminiumoxidwirbelschicht einbringt und unter Mitverwendung von Wasserdampf bei einer Temperatur von 290 bis 400°C spaltet bekannt. Es hat sich jedoch herausgestellt, daß diese Arbeitsweise zu Verstopfungen in den Brüdenleitungen führt, die häufige Betriebsunterbrechungen erforderlich machen.

In der DE-A 26 41 381 wird die katalytische Umlagerung von Cyclohexanonoxim mit Boroxid auf Aluminiumoxid in der Wirbelschicht beschrieben. Ferner wird in der DE-A 19 53 153 eine katalytische Umlagerung von Cyclohexanonoxim in Gegenwart von Boroxid-Aluminiumoxidkatalysatoren nach dem Wirbelfließverfahren beschrieben, bei dem die katalytische Umlagerung in der Wirbelschicht durchgeführt wird, der mit Crackprodukten beladene Katalysator durch eine Rohrleitung in ein Regeneriergefäß geleitet wird und der regenerierte Katalysator zurückgeführt wird. Dem aus der Wirbelschicht abgezogenen Katalysator wird in dem Rohr zum Regeneriergefäß Stickstoff entgegengeführt um ein Verbacken zu vermeiden.

Es war deshalb die technische Aufgabe gestellt, ein Verfahren zur Spaltung von Oligomeren des Caprolactams in einer Aluminiumoxidwirbelschicht zur Verfügung zu stellen, bei der keine Verstopfungen der Brüdenleitungen und damit verbundene häufige Unterbrechungen eintreten.

Diese Aufgabe wird gelöst in einem Verfahren zur Gewinnung von Caprolactam durch Spaltung von Oligomeren des Caprolactams, wobei man die Oligomeren flüssig oder in festem Zustand in eine Aluminiumoxidwirbelschicht einbringt und unter Mitverwendung von 0.05 bis 10 Gew. Teilen Wasserdampf je Gew.Teil Oligomere bei einer Temperatur von 290 bis 400°C unter Einhaltung einer Verweilzeit von 0.1 bis 30 Sekunden spaltet, dadurch gekennzeichnet, daß man oberhalb der Wirbelschicht die 0,1 bis 3-fache Inertgasmenge zusätzlich zu derjenigen einbringt, wie sie zum Wirbeln der Aluminiumoxidschicht benötigt wird.

Das neue Verfahren hat den Vorteil, daß praktisch keine Verstopfungen mehr eintreten und das Verfahren über einen langen Zeitraum ohne Unterbrechung ausgeübt werden kann. Ferner hat das neue Verfahren den Vorteil, daß der Abrieb an der Aluminiumoxidwirbelschicht wesentlich vermindert wird.

Oligomere, die als Ausgangsstoffe eingesetzt werden, haben in der Regel einen Polymerisationsgrad n von 2 bis 9. Insbesondere enthalten sie dimere und trimere cyclische Oligomere. Solche Oligomere werden beispielsweise erhalten durch Eindampfen von Waschwässern, die bei der Extraktion von Polycaprolactam anfallen und anschließende Entfernung des monomeren Caprolactams durch Destillation. Vorteilhaft wendet man die Oligomeren im Gemisch mit Caprolactam an. Es ist deshalb nicht erforderlich, aus dem anfallenden Extrakt die gesamte Menge Caprolactam abzudestillieren. Geeignete Gemische enthalten beispielsweise 10 bis 60 Gew.% Oligomere und 90 bis 40 Gew.% Caprolactam. Die Oligomeren oder die Gemische aus Oligomeren und Caprolactam werden vorteilhaft in flüssiger Form, d.h. in geschmolzenem Zustand z.B. mit einer Temperatur von 150 bis 250°C in ein Aluminiumoxidwirbelbett eingebracht. Es ist jedoch auch möglich, die Oligomeren bzw. das Oligomeren-Lactamgemisch in fester feinverteilter Form in die Wirbelschicht einzubringen und katalytisch in monomeres Caprolactam zu spalten. Das Einbringen in die Wirbelschicht erfolgt z.B. durch Einblasen mittels einer mit Inertgas betriebenen Düse.

Als Aluminiumoxid eignen sich die verschiedenen Modifikationen, wie Tonerde oder Böhmit, besonders bewährt hat sich γ-Aluminiumoxid als Katalysator. Der Katalysator wird mit Inertgas wie Kohlendioxid, Argon oder Stickstoff, vorzugsweise Stickstoff in wirbelnder Bewegung gehalten. Zweckmäßig verwendet man Aluminiumoxid in Korngrößen von 0,05 bis 1,5 mm, insbesondere von 0,2 bis 1 mm. Die Höhe der Katalysatorschicht wird so gewählt, daß die Verweilzeiten der Oligomeren an der Katalysatorschicht von 0,1 bis 30, insbesondere von 0,5 bis 10 sek. betragen. Vorteilhaft führt man das Verfahren bei Normaldruck durch. Es kann jedoch auch bei schwach vermindertem oder leichtem Überdruck, z.B. bis zu 2 bar durchgeführt werden.

Die Katalysatorschicht wird auf einer Temperatur von 290 bis 400°C, insbesondere 300 bis 360°C gehalten. Es ist deshalb auch zweckmäßig, das Inertgas mit einer Temperatur von 290 bis 400°C der Wirbelschicht zuzuführen.

Ein wesentliches Merkmal der Erfindung ist es, daß man Inertgas nicht nur in die Wirbelschicht einleitet, zum Zwecke des Verwirbeln von Aluminiumoxid bzw. zum Einbringen der gespaltenen Oligomeren, sondern daß man zusätzlich oberhalb der Wirbelschicht eine weitere Menge Inertgas zuführt. Erfindungsgemäß führt man deshalb oberhalb der Wirbelschicht die 0,1 bis 3-fache Inertgasmenge zusätzlich zu derjenigen zu, wie sie zum Wirbeln der Aluminiumoxidschicht benötigt wird. Es hat sich ferner als vorteilhaft erwiesen, wenn man in der Wirbelschicht eine Wirbelgeschwindigkeit von 10 bis 40 cm/sek., bezogen auf den freien Querschnitt einhält.

Vorteilhaft hält man im Gasraum oberhalb der Wirbelschicht einen Caprolactampartialdruck von 50 bis 300 mbar ein.

Die Spaltung führt man unter Mitverwendung von Wasserdampf durch. Man verwendet man je Gewichtsteil Oligomere 0,005 bis 10 Gew.-Teile, insbesondere 0,02 bis 2 Gew.-Teile Wasser in Form von Wasserdampf. Das mitzuverwendende Wasser kann als solches in die Wirbelschicht eingebracht und dort verdampft werden. Vorzugsweise führt man das Wasser jedoch in Form von Wasserdampf zu. Beispielsweise kann dieser auch zusammen mit dem Inertgas in die Wirbelschicht eingeführt werden.

Das aus der Wirbelschicht austretende Gasgemisch wird wie z.B. in der DE-AS 14 45 549 beschrieben, in einer Glockenbodenkolonne durch Aufgabe von Wasser auf den Kopf der Kolonne kondensiert. Als Sumpfprodukt erhält man Caprolactam. Über Kopf der Kolonne entweichen das Inertgas sowie Wasserdampf. Der Wasserdampf kann aus dem Inertgas kondensiert werden und das Inertgas wird zweckmäßig wieder in die Wirbelschicht und oberhalb der Wirbelschicht zurückgeführt.

Das kondensierte Caprolactam kann z.B. durch Destillation nochmals gereinigt werden und anschließend das so zurückgewonnene Caprolactam dem zu reinigenden Caprolactam aus der Beckmann'schen Umlagerung zugesetzt und gereinigt werden, wie beispielsweise in der DE-PS 1 194 863 beschrieben.

Es ist aber auch möglich, das Caprolactam aus dem Reaktor austretenden Dämpfegemisch wie beschrieben zu kondensieren und diese direkt dem Rohlactam aus der Beckmann'schen Umlagerung zuzufügen und gemeinsam aufzuarbeiten.

Caprolactam wird für die Herstellung von Polycaprolactam verwendet. Das Verfahren nach der Erfindung sei an folgenden Beispielen veranschaulicht.

## Vergleichsbeispiel

In einem senkrecht stehenden Reaktor von 1000 mm Durchmesser und einem Durchmesser des über der Wirbelschicht befindlichen Gasraumes von 1500 mm wird eine Wirbelschicht aus 1200 kg Aluminiumoxid aufrechterhalten. Die Korngröße des Aluminiumoxids beträgt durchschnittlich 200 bis 500 µm. Das Wirbeln des Katalysators wird durch Einblasen eines vorgeheizten Stickstoffstromes von 300 kg/Stunde, der von unten durch einen Lochboden geleitet wird, bewirkt. Dem Stickstoff werden stündlich 60 kg Wasserdampf zugeführt. In der Wirbelschicht wird eine Temperatur von 290 bis 300°C aufrechterhalten.

Durch 2 Düsen, welche um 180°C versetzt über den Umfang des Reaktors verteilt und ca. 500 mm über dem Lochboden angeordnet sind, werden stündlich 400 kg eines Gemisches aus Caprolactam und Oligomeren mit einem Oligomerengehalt von 12 Gew.% durch Verdüsen mit 30 kg Stickstoff/Stunde und Düse eingebracht. Die Wirbelgeschwindigkeit bezogen auf den freien Querschnitt beträgt 29,6 cm/sek. Der Caprolactampartialdruck beträgt 199,7 mbar. Die Verweilzeit beträgt 6,4 sek.

Das den Reaktor verlassende Dämpfegemisch gelangt nach Durchströmen eines Zyklons zur Abscheidung des Katalysatorabriebs in eine Kolonne, in der das Caprolactam durch Aufgabe von Wasser auf den Kolonnenkopf kondensiert wird. Auf diese Weise werden stündlich 396,3 kg Caprolactam mit einem Restoligomerengehalt von ungefähr 0,1 Gew.% erhalten. Bei dieser Arbeits weise beträgt der Katalysatorabrieb 23 kg/Tag. Nach einem Betrieb von 6 Wochen zeigten sich in den Brüdenleitungen soviel Ablagerungen, daß sie mechanisch entfernt werden mußten. Der Caprolactampartialdruck in der Brüdenleitung betrug 199,7 mbar.

## Beispiel

Wie im Vergleichsbeispiel beschrieben, werden 400 kg eines Gemisches aus Caprolactam und Oligomeren mit einem Gehalt von 12 Gew.% Oligomeren in die Wirbelschicht in gleicher Höhe wie im Vergleichsbeispiel mit 30 kg Stickstoff/Stunde und Düse eingebracht. Die zum Wirbeln des Katalysators verwendete Stickstoffmenge beträgt jedoch nur 160 kg/Stunde. Die mit dem Stickstoffstrom in den Reaktor eingebrachte Wasserdampfmenge beträgt 60 kg/Stunde. In der Wirbelschicht wird eine Temperatur von 290 bis 300°C aufrechterhalten. Oberhalb der Wirbelschicht werden in den Gasraum des Reaktors zusätzlich 200 kg/Stunde Stickstoff eingebracht.

Die Wirbelgeschwindigkeit, bezogen auf den freien Querschnitt, beträgt 22,1 cm/sek. Der Caprolactampartialdruck beträgt 267,5 mbar. Die Verweilzeit beträgt 8,6 sek. Aus den Brüden werden stündlich 396,6 kg Caprolactam mit einem Restoligomerengehalt von ungefähr 0,1 Gew.% erhalten.

Bei dieser Arbeitsweise werden auch nach einer Zeit von 16 Wochen keine Ablagerungen in der Brüdenleitung festgestellt. Der Caprolactpartialdruck in der Brüdenleitung betrug 180,1 mbar.

## Ansprüche

1. Verfahren zur Gewinnung von Caprolactam durch Spaltung von Oligomeren des Caprolactams, wobei man die Oligomeren flüssig oder in festem Zustand in eine Aluminiumoxidwirbelschicht einbringt und unter Mitverwendung von 0,05 bis 10 Gew. Teilen Wasserdampf je Gew. Teil Oligomere bei einer Temperatur von 290 bis 400°C unter Einhaltung einer Verweilzeit von 0.1 bis 30 Sekunden spaltet, dadurch gekennzeichnet, daß man oberhalb der Wirbelschicht die 0,1 bis 3-fache Inertgasmenge zusätzlich zu derjenigen einbringt, wie zum Wirbeln der Aluminiumoxidschicht benötigt wird.

2. Verfahren nach Anspruch 1, dadurch gekennzeichnet, daß man in der Wirbelschicht eine Wirbelgeschwindigkeit von 10 bis 40 cm/sek., bezogen auf den freien Querschnitt, einhält.

3. Verfahren nach den Ansprüchen 1 und 2, dadurch gekennzeichnet, daß man im Gasraum oberhalb der Wirbelschicht einen Caprolactampartialdruck von 50 bis 300 mbar einhält.

## Claims

1. A process for obtaining caprolactam by cleaving oligomers of caprolactam, wherein the oligomers, in a liquid or solid state, are introduced into a fluidized alumina bed and cleaved at from 290 to 400°c in the presence of from 0.05 to 10 parts by weight of steam per part by weight of oligomers for a residence time of from 0.1 to 30 seconds, wherein, in addition to the amount of inert gas required to fluidize the alumina bed, from 0.1 to 3 times this amount of inert gas is introduced above the fluidized bed.

2. A process as claimed in claim 1, wherein a fluidization velocity of from 10 to 40 cm/sec, based on the free cross-section, is maintained in the fluidized bed.

3. A process as claimed in either of claims 1 and 2, wherein a caprolactam partial pressure of from 50 to 300 mbar is maintained in the gas space above the fluidized bed.

## Revendications

1. Procédé d'obtention de caprolactame par décomposition d'oligomères de caprolactame, où on introduit les oligomères à l'état liquide ou à l'état solide dans un lit fluidisé d'oxyde d'aluminium et on décompose avec emploi simultané de 0,05 à 10 parties en poids de vapeur d'eau par partie en poids d'oligomères à une température de 290 à 400°C en maintenant un temps de séjour de 0,1 à 30 s, caractérisé en ce qu'on introduit au dessus du lit fluidisé une quantité supplémentaire de gaz inerte qui est 0,1 à 3 fois celle nécessaire pour fluidiser le lit d'oxyde d'aluminium.

2. Procédé selon la revendication 1, caractérisé en ce qu'on maintient dans le lit fluidisé une vitesse de fluidisation de 10 à 40 cm/s, par rapport à la section libre.

3. Procédé selon l'une ou l'autre des revendications 1 et 2, caractérisé en ce qu'on maintient dans la chambre à gaz au dessus du lit fluidisé une pression partielle de caprolactame de 50 à 300 mbar.